# EUROPEAN PATENT SPECIFICATION

(11) **EP 3 012 035 B1**
(45) Date of publication and mention of the grant of the patent: **24.04.2019**
(21) Application number: 14187165.7
(22) Date of filing: 30.09.2014
(51) Int. Cl.: B08B 3/02, B08B 3/00, A61B 1/12, A61B 90/70

(54) **Cleaning device**
Reinigungsvorrichtung
Dispositif de nettoyage

(43) Date of publication of application: 27.04.2016
(73) Proprietor: Renato Polinari de Araujo, Paraná (BR)
(72) Inventor: Renato Polinari de Araujo, 81.200-110 Curitiba Paraná (BR); FAUSTO, Fernandes Hilario Gomes, 81110-10 Curitiba-Paraná (BR)
(74) Representative: Pfenning, Meinig & Partner mbB

(56) References cited:
- JP-A- 2000 166 869
- US-A- 2 571 575
- US-A1- 2005 241 685
- US-A1- 2009 133 721
- US-B1- 6 199 293

## Description

The present invention relates to a cleaning device comprising one inlet and one outlet for air, one inlet and one outlet for water, one inlet and one outlet for at least one chemical agent and a dosing system for the at least one chemical agent.

Within health institutions such as clinics, hospitals, diagnostic centres or dental centres, cleaning devices are used for cleaning and disinfection.

US 6,199,293 B1 discloses a cleaning device in accordance with the preamble of claim 1.

Cleaning devices known from the prior art have several problems:
- Lack of repeatability in relation to stream,
- Risk of contact with concentrated sanitizing chemicals,
- Difficulty of fixing or docking of lines or catheters during the process of cleaning and sterilization.

Thus, it is the object of the present invention to provide an improved cleaning device which overcomes the above-mentioned problems of the prior art devices.

This object is achieved by the cleaning device according to claim 1. Claim 16 is directed to the use of the cleaning device according to the invention. Further advantageous embodiments are contained in the dependent claims.

According to the invention, a cleaning device comprising one inlet and one outlet for air, one inlet and one outlet for water, one inlet and one outlet for at least one chemical agent and a dosing system for the at least one chemical agent is provided. The outlet for air, the outlet for water and the outlet for the at least one chemical agent are pressurized pistols. The outlet for the at least one chemical agent is connected to the inlet for the at least one chemical agent and the inlet for water via a valve such that a dilution of the at least one chemical agent with water can be discharged via the outlet for the at least one chemical agent. The device is designed such that after entering a desired concentration of the at least one chemical agent in the discharged dilution to the device, the position of the valve is adjusted by the dosing system automatically such that the discharged dilution has the desire concentration.

The cleaning device according to the present invention assists the cleaning process by combining the action of the water jets associated or not to a cleaning agent, dismissed by their pistols. The cleaning device is endowed with three pistols, namely: chemical agent to aid cleaning, rinsing water and air for drying.

Air passes into the device via the inlet for air and is discharged via the outlet for air, i.e. the pressurized pistol for air. Water passes into the device via the inlet for water and may be discharged via the outlet for water, i.e. the pressurized pistol for water. The at least one chemical agent passes into the device via the inlet for the at least one chemical agent and may be discharged via the outlet for the at least one chemical agent, i.e. the pressurized pistol for the at least one chemical agent. Furthermore, the outlet for the at least one chemical agent is connected to the inlet for the at least one chemical agent and the inlet for water via a valve such that also a dilution of the at least one chemical agent with water can be discharged via the outlet for the at least one chemical agent. This dilution has a desired concentration of the at least one chemical agent since the dosing system adjusts this desired concentration after entering it to the device e.g. via operating buttons.

The cleaning device according to the invention has several advantages:
- The cleaning device has three pressurized pistols (one for water, one for air and one for a chemical agent) in the same body which facilitates the process for the operator and for the installation.
- By using pressurized pistols for water, air and the chemical agent, it is possible to work with controlled and stable pressure assuring repeatability in the process.
- It is possible to provide the at least one chemical agent already diluted and homogenized in programmed concentration from a gallon of concentrated chemical agent automatically.
- The variable dosing system adapts to the concentration recommended for a chemical agent such as cleaning agents, allowing simultaneous application in recommended concentration, avoiding waste of product and dosage errors.
- The cleaning device may run low dosages with extreme precision.
- The cleaning device aspires and doses the chemical agent automatically without the need for operator intervention and thus exposing the operator as little as possible to concentrated chemicals.
- With respect to the flow, the equipment maintains the same always controlled which considerably increases the efficiency of the cleaning process.

In a preferred embodiment of the cleaning device according to the present invention, the chemical agent is a cleaning agent, preferably a cleaning agent for medical purposes. This cleaning agent can e.g. be a cleaning agent with lipolytic and/or proteolytic and/or disinfectant effect.

A further preferred embodiment of the cleaning device according to the present invention is characterized in that the desired concentration can be entered to the device via operating buttons arranged at the device.

In a further preferred embodiment, the specific concentration can be achieved by diluting the at least one chemical agent with water. The ratio of dilution with water can preferably be in between 1:99 to 1:1, preferably 1:50 to 1:5.

Furthermore, it is preferred that the water discharged via the outlet for water has a temperature of < 50 °C and/or is discharged with a pressure from 0,1 MPa to 0,5 MPa.

A further preferred embodiment is characterized in that the air discharged via the outlet for air has a pressure of ≤ 0,5 MPa.

In a further preferred embodiment, the pressurized pistols are with or without pressure control. With this embodiment it is possible to deliver air with or without pressure control. Hence, the user can leave a fixed pressure or set the same in the case of sensitive materials drying. Furthermore, it is possible to provide water with or without pressure control. Thus, the user can leave a water pressure fixed or set the same in the case of delicate materials or rinse to avoid "sprinkling" of water during the rinsing process. This is very useful in controlling the spread of contaminants. Moreover, it is possible to provide a dilution of the chemical agent with or without pressure control, where the user can leave a water pressure fixed or set the same in the case of delicate materials or rinse to avoid "sprinkling" of water during the rinsing process. This is very useful in controlling the spread of contaminants.

Furthermore, it is preferred that each pressurized pistol comprises an electronic button that is positioned on the head of the pressurized pistol. The device is preferably designed such that the electronic button has to be touched once to activate the corresponding pressurized pistol and once more to deactivate it. The user triggers the equipment with just a touch and shuts it off with another. Thus, there is no need to keep the button pressed. The firings of the guns are the same and are easily activated with a simple touch and remain activated until the next touch.

A further preferred embodiment is characterized in that each pressurized pistol is essentially made of aluminium. This preferred embodiment is lightweight and has greater resistance.

In a further preferred embodiment, each pressurized pistol comprises at least one tip. The at least one tip is preferably made of stainless steel and silicone. Such tip does not deform due to the stainless steel and the silicone stopper prevents spills.

Furthermore, it is preferred that the at least one tip is a luer slip tip. This luer slip tip is preferably tapered. Such tip fits perfectly to various types of connection.

A further preferred embodiment is characterized in that the tip is interchangeable. The tip can preferably be replaced by a tip with multiple applications and/or with an integrated shower-head function. The shower-head function provides the best rinse of external materials. The "showerhead" is an interchangeable tip which can be overridden in the levers (guns) to expand the applications with this device. Instead of a concentrated jet, the user will have a shower which facilitates application of product or rinse over large areas. Multiple applications may be inject products from an endoscope to a catheter.

In a further preferred embodiment, the device according to the invention comprises a display, preferably an LCD display, a Touch display or an LCD-Touch display.

Furthermore, it is preferred that the device according to the invention comprises a peristaltic pump, preferably a peristaltic pump with a stepper motor. Thus, there is no difficulty of accuracy of dosage and chemical product flow.

In a further preferred embodiment, the device according to the invention comprises at least one safety system that protects against possible failures, such as chemical or water shortage. Such system may also monitor the amount of water that passes through the dosing system and may check whether it is exactly the requested amount that has been entered or programmed.

The cleaning device according to the invention can be used in processes of solution preparation, dosing, cleaning and/or disinfection in health institutions, clinics, hospitals, diagnostic centres and/or dentals centres.

With the cleaning device according to the present invention, it is possible to programme several dosages, i.e. concentrations of chemical agent in the diluent. Thus, it is not necessary to enter the specific value of a concentration to the device before discharging a diluent with this concentration.

In addition, the cleaning device preferably has an active product conference system which guarantees safety against incorrect use.

The cleaning device may have an additional inlet for alcohol. An aerosol of alcohol and air may be discharged via the outlet for air in order to improve drying.

The cleaning device was developed to be used in clinics, hospitals, diagnostic centres, dental centres among others. The cleaning device device assists the cleaning process by combining the action of the water jets associated or not to a cleaning agent, dismissed by their pistols. The variable dosing system of the cleaning device adapts to the concentration recommended for cleaning agents, allowing simultaneous application in recommended concentration, avoiding waste of product and dosage errors. The cleaning device is endowed with three pistols, namely: chemical agent to aid cleaning, rinsing water and air for drying. Features activation button, positioned strategically on head of pistols, allowing the user quick handling of the same. The guns of the cleaning device with stainless steel and silicone tips adapting to different types of cannulated items or lumens.

The Tip-DS dosing system adds the functionality to use in luer lock connections, and may in addition use already described in patent pending adapt to catheters s bloodlines to run their respective cleaning and fills with saneante solution.

This peristaltic pump with stepper motor is designed to increase the accuracy of equipment developed by the company, which require a high level of precision of dosage, such as the DS and the cleaning device among others, with a view in the case of a peristaltic pump exclusively.

The professional can count on three pressurized pistols in a single device, one that injects the cleaning or disinfecting agent dosed in concentration automatically programmed, because it has a built-in feeder DS type system, and two other pistols of water to rinse and air for drying by making the equipment a versatile tool and unique on the market.

Improvement of tip in this new DS system account with luer lock tip where the user can connect various types of catheters or tubes that have this device to perform cleaning or disinfecting.

The equipment of the cleaning device provides through its water pistols, chemist or air with controlled and stable pressure assuring repeatability in the process. With respect to the flow the equipment maintains the same always controlled what considerably increases the efficiency of the cleaning process. The equipment has intelligent system (SGPU) patented in DS, where performs the dosing automatically programmed without risk of human error, this system also avoids variations, such as blisters or sudden changes of pressure in water input automatically adjusting the dosage. When connecting the cleaning device tip of the cannulated has stainless steel which does not deform and fits perfectly to various types of connection due to its luer slip tip (tapered) and silicone stopper that prevents spills in the professional. The pistol of the equipment has been designed to be the most ergonomic possible lightweight for being superficial treatment with aluminum, which urged greater resistance, his drive takes place through electronic button positioned on the same, where the Professional with just a touch triggers the equipment and another to shut it off there is no need to keep it fires. The appliance has 3 pistol (chemical, water and air) in the same body which facilitates the process for the operator and for the installation. The cleaning device system aspires and dose chemical scheduled automatically without the need for operator intervention exposing as little as possible to concentrated chemicals. The equipment allows the operator to several programmable dosages which after selected on the display (LCD) will be executed automatically. The equipment has safety systems that protects against possible failures, such as lack of chemical or water shortage, also monitors the amount of water that passes through the dosing equipment exactly the requested by programming.
In addition to the items above mentioned security equipment has active product conference system and presence of pistol which guarantees safety against incorrect use. For rinse products pre washed the equipment account with "shower head" shower which provides the best rinse the external materials. The tip DS solved the problem of luer type connection of catheters and blood lines. The peristaltic pump solved the difficulty of accuracy of dosage and chemical product flow.

The cleaning device is a versatile equipment "semi-automated" processes within the hospital environment that delivers air with or without pressure control where the operator can leave a fixed pressure or set the same in the case of sensitive materials drying. Provides water with or without pressure control where the operator can leave a water pressure fixed or set the same in the case of delicate materials or rinse to avoid "sprinkling" of water during the rinsing process. This is very useful in controlling the spread of contaminants. Provides chemical (sanitizing in General) already diluted and homogenized in programmed concentration from a gallon of concentrated product automatically, this system of dosage is accurate and may run low dosages and with extreme precision (even the DS system), still provides the solution with or without pressure control (single market) where the operator can leave a water pressure fixed or set the same in the case of delicate materials or rinse to avoid "sprinkling" of water during the rinsing process. This is very useful in controlling the spread of contaminants.

The cleaning device also features a device known as "showerhead" (shower) which is an interchangeable tip which can be overridden in the levers (guns) to expand the applications with this device instead of a concentrated jet operator will have a shower which facilitates application of product or rinse over large areas.

All the tips of pistols of the equipments are interchangeable and can have multiple applications such as inject product from an endoscope to a catheter because the tips are luer slip and all are extremely resistant stainless steel and easy to be sanitized.

The firing of the guns are the same and are easily activated with a simple touch and remains activated until the next touch.

The equipment has a device of "rinsing with alcohol", which can when enabled in the Panel releases a jet of alcohol with air in air pistol.

This equipment is automatic bivolt which facilitates the use in all areas.

In your dashboard (display) may accompany each process simultaneously, because the equipment has sensors that identify which gun is being used.

The equipment also has a security system that informs the operator when the lack of water or product end stopping the process.

The system there is also the "leak test" function used to test checks the integrity of endoscopes optional.

The cleaning device has tips in their pistols which are interchangeable serve a variety of applications. (e.g., wash, rinse, dry, injecting liquids in tubes or catheters, etc.), they are made of stainless steel which prints an extremely high durability. The pistol-firing takes place in own pistol which facilitates the use of the same, not needing to be pressed for time of use and Yes, just a touch to connect and a touch to switch to terminate the process, representing a substantial ergonomics for the user.

The equipment can be made available in two versions, with or without pressure control in their pistols, so the operator can count on a fixed pressure or with a version where the same can program the pressure or select operation where pre-pressure sets, depending on use.

The device of rinsing with alcohol is optional and can be used by the operator in cases where the same requires a more effective drying. In this case the diluted alcohol is expelled by the medicinal air pistol intensifying the drying.

The Panel is also found in two versions, with LCD display or Touch depending on the version selected by the client, if the equipment display tera a membrane keypad for programming otherwise touch version program-if straight on the screen. This device serves as well as informative programming where the operator can obtain various information, such as: Operation pressure (water, air, saneante), stream, dosage, time and what operation is being performed as well as the name of the professional who is running the process at the moment.

The leak test is an optional available for professionals in endoscopes reprocessam the cleaning device, this is a device that tests the integrity of the endoscope prior to reprocessing, to avoid possible damage to the same. The result of this test appears on the display or the screen informing you if the endoscope is fit to reuse or requires repairs.

The equipment also features system that identify which scope is being reprocessed, which is the operation and the operator running procedure and sent the data to a central.

The cleaning device may be willing to inject more than one type of saneante which facilitated its use (only one on the market).

The device may send the data process and user who ran to a central and the central provides this data to the client.

The cleaning device has in its Constitution a DS system (with SGPU system) which provides a precision variation independent of water pressure at the entrance and will own the content identification system of the active substance in the product produced. The equipment still has an exclusive design for easy sanitization.

The proposed equipment will be used in the processes of solution preparation, dosing, cleaning and disinfection through the use of chemicals within health institutions.

## Claims

1. Cleaning device comprising one inlet and one outlet for air, one inlet and one outlet for water, one inlet and one outlet for at least one chemical agent and a dosing system for the at least one chemical agent,
charactericed in that the cleaning device has three pressurised pistols, wherein the outlet for air is a pressurized pistol for air, the outlet for water is a pressurized pistol for water and the outlet for the at least one chemical agent is a pressurized pistol for the at least one chemical agent,
wherein the outlet for the at least one chemical agent is connected to the inlet for the at least one chemical agent and the inlet for water via a valve such that a dilution of the at least one chemical agent with water can be discharged via the outlet for the at least one chemical agent,
wherein the device is designed such that after entering a desired concentration of the at least one chemical agent in the discharged dilution to the device, the position of the valve is adjusted by the dosing system automatically such that the discharged dilution has the desired concentration.

2. Cleaning device according to claim 1, **characterized in that** the chemical agent is a cleaning agent, preferably a cleaning agent for medical cleaning purposes.

3. Cleaning device according to one of the preceding claims, **characterized in that** the desired concentration can be entered to the device via operating buttons arranged at the device.

4. Cleaning device according to one of the two preceding claims, **characterized in that** the dilution of the at least one chemical agent with water can be adjusted in between 1:99 to 1:1, preferably 1:50 to 1:5.

5. Cleaning device according to one of the preceding claims, **characterized in that** the water discharged via the outlet for water has a temperature of < 50 °C and/or is discharged with a pressure from 0,1 MPa to 0,5 MPa.

6. Cleaning device according to one of the preceding claims, **characterized in that** the air discharged via the outlet for air has a pressure of ≤ 0,5 MPa.

7. Cleaning device according to one of the preceding claims, **characterized in that** the pressurized pistols are with or without pressure control.

8. Cleaning device according to one of the preceding claims, **characterized in that** each pressurized pistol comprises an electronic button that is positioned on the head of the pressurized pistol and that the device is preferably designed such that the electronic button has to be touched once to activate the corresponding pressurized pistol and once more to deactivate it.

9. Cleaning device according to one of the preceding claims, **characterized in that** each pressurized pistol is essentially made of aluminium.

10. Cleaning device according to one of the preceding claims, **characterized in that** each pressurized pistol comprises at least one tip, wherein the at least one tip is preferably made of stainless steel and silicone.

11. Cleaning device according to the preceding claim, **characterized in that** the at least one tip is a luer slip tip that is preferably tapered.

12. Cleaning device according to one of the two preceding claims, **characterized in that** the tip is interchangeable and can preferably be replaced by a tip with multiple applications and/or with an integrated shower-head function,

13. Cleaning device according to one of the preceding claims, **characterized in that** it comprises a display, preferably an LCD display, a Touch display or an LCD-Touch display.

14. Cleaning device according to one of the preceding claims, **characterized in that** it comprises a peristaltic pump, preferably a peristaltic pump with a stepper motor.

15. Cleaning device according to one of the preceding claims, **characterized in that** it comprises at least one safety system that protects against possible failures, such as chemical or water shortage.

16. Use of a cleaning device according to one of the preceding claims in processes of solution preparation, dosing, cleaning and/or disinfection in health institutions, clinics, hospitals, diagnostic centres and/or dentals centres.

## Patentansprüche

1. Reinigungsgerät, umfassend einen Einlass und einen Auslass für Luft, einen Einlass und einen Auslass für Wasser, einen Einlass und einen Auslass für mindestens ein chemisches Mittel und ein Dosiersystem für das mindestens eine chemische Mittel,
**dadurch gekennzeichnet, dass**
das Reinigungsgerät drei Druckpistolen aufweist, wobei der Auslass für Luft eine Druckpistole für Luft ist, der Auslass für Wasser eine Druckpistole für Wasser ist und der Auslass für das mindestens eine chemische Mittel eine Druckpistole für das mindestens eine chemische Mittel ist,
wobei der Auslass für das mindestens eine chemische Mittel mit dem Einlass für das mindestens eine chemische Mittel und mit dem Einlass für Wasser über ein Ventil verbunden ist, so dass eine Lösung des mindestens einen chemischen Mittels mit Wasser über den Auslass für das mindestens eine chemische Mittel abgeführt werden kann,
wobei das Gerät so ausgeführt ist, dass nach dem Eingeben einer gewünschten Konzentration des mindestens einen chemischen Mittels in der abgeführten Lösung in das Gerät, die Position des Ventils durch das Dosiersystem automatisch eingestellt wird, so dass die abgeführte Lösung die gewünschte Konzentration aufweist.

2. Reinigungsgerät gemäß Anspruch 1, **dadurch gekennzeichnet, dass** das chemische Mittel ein Reinigungsmittel, bevorzugt ein Reinigungsmittel für medizinische Reinigungszwecke, ist.

3. Reinigungsgerät gemäß einem der vorhergehenden Ansprüche, **dadurch gekennzeichnet, dass** die gewünschte Konzentration über an dem Gerät angeordnete Knöpfe in das Gerät eingegeben werden kann.

4. Reinigungsgerät gemäß einem der zwei vorhergehenden Ansprüche, **dadurch gekennzeichnet, dass** die Lösung des mindestens einen chemischen Mittels mit Wasser zwischen 1:99 und 1:1, bevorzugt zwischen 1:50 und1:5, eingestellt werden kann.

5. Reinigungsgerät gemäß einem der vorhergehenden Ansprüche, **dadurch gekennzeichnet, dass** das über den Auslass für Wasser abgeführte Wasser eine Temperatur von < 50 °C aufweist und/oder mit einem Druck von 0,1 MPa bis 0,5 MPa abgeführt wird.

6. Reinigungsgerät gemäß einem der vorhergehenden Ansprüche, **dadurch gekennzeichnet, dass** die über den Auslass für Luft abgeführte Luft einen Druck von ≤ 0,5 MPa aufweist.

7. Reinigungsgerät gemäß einem der vorhergehenden Ansprüche, **dadurch gekennzeichnet, dass** die Druckpistolen mit oder ohne Drucksteuerung sind.

8. Reinigungsgerät gemäß einem der vorhergehenden Ansprüche, **dadurch gekennzeichnet, dass** jede Druckpistole einen elektronischen Knopf umfasst, der am Kopf der Druckpistole positioniert ist, und dass das Gerät vorzugsweise so ausgeführt ist, dass der elektronische Knopf einmal berührt werden muss, um die entsprechende Druckpistole zu aktivieren, und noch einmal, um sie zu deaktivieren.

9. Reinigungsgerät gemäß einem der vorhergehenden Ansprüche, **dadurch gekennzeichnet, dass** jede Druckpistole im Wesentlichen aus Aluminium hergestellt ist.

10. Reinigungsgerät gemäß einem der vorhergehenden Ansprüche, **dadurch gekennzeichnet, dass** jede Druckpistole mindestens eine Spitze umfasst, wobei die mindestens eine Spitze vorzugsweise aus rostfreiem Stahl und Silikon hergestellt ist.

11. Reinigungsgerät gemäß dem vorhergehenden Anspruch, **dadurch gekennzeichnet, dass** die mindestens eine Spitze eine Luer-Slip-Spitze ist, die vorzugsweise verjüngt ist.

12. Reinigungsgerät gemäß einem der zwei vorhergehenden Ansprüche, **dadurch gekennzeichnet, dass** die Spitze austauschbar ist und vorzugsweise durch eine Spitze mit mehreren Anwendungen und/oder mit integrierter Duschkopffunktion ersetzt werden kann.

13. Reinigungsgerät gemäß einem der vorhergehenden Ansprüche, **dadurch gekennzeichnet, dass** es ein Display, bevorzugt ein LCD-Display, ein Touch-Display oder ein LCD-Touch-Display, umfasst.

14. Reinigungsgerät gemäß einem der vorhergehenden Ansprüche, **dadurch gekennzeichnet, dass** es eine Schlauchpumpe, bevorzugt eine Schlauchpumpe mit einem Schrittmotor, umfasst.

15. Reinigungsgerät gemäß einem der vorhergehenden Ansprüche, **dadurch gekennzeichnet, dass** es mindestens ein Sicherheitssystem umfasst, dass gegen mögliche Fehler, wie Chemikalien- oder Wassermangel, absichert.

16. Verwendung eines Reinigungsgeräts gemäß einem der vorhergehenden Ansprüche in Verfahren zur Lösungsherstellung, Dosierung, Reinigung und/oder Desinfektion in Krankenanstalten, Kliniken, Krankenhäusern, Diagnosezentren und/oder Dentalzentren.

## Revendications

1. Dispositif de nettoyage comprenant une entrée et une sortie pour l'air, une entrée et une sortie pour l'eau, une entrée et une sortie pour au moins un agent chimique et un système doseur pour l'au moins un agent chimique,
**caractérisé en ce que**
le dispositif de nettoyage comporte trois pistolets sous pression, la sortie pour l'air étant un pistolet sous pression pour l'air, la sortie pour l'eau étant un pistolet sous pression pour l'eau et la sortie pour l'au moins un agent chimique étant un pistolet sous pression pour l'au moins un agent chimique,
dans lequel la sortie pour l'au moins un agent chimique est raccordée à l'entrée pour l'au moins un agent chimique et l'entrée pour l'eau par l'intermédiaire d'une vanne de sorte qu'une dilution de l'au moins un agent chimique avec l'eau puisse être évacuée par la sortie pour l'au moins un agent chimique,
le dispositif étant conçu de sorte qu'après l'entrée d'une concentration souhaitée de l'au moins un agent chimique dans la dilution évacuée dans le dispositif, la position de la vanne est automatiquement ajustée par le système doseur de sorte que la dilution évacuée ait la concentration souhaitée.

2. Dispositif de nettoyage selon la revendication 1, **caractérisé en ce que** l'agent chimique est un agent de nettoyage, de préférence un agent de nettoyage pour des applications de nettoyage médicales.

3. Dispositif de nettoyage selon l'une des revendications précédentes, **caractérisé en ce que** la concentration souhaitée peut être entrée dans le dispositif par l'intermédiaire de boutons opérationnels agencés sur le dispositif.

4. Dispositif de nettoyage selon l'une des deux revendications précédentes, **caractérisé en ce que** la dilution de l'au moins un agent chimique avec de l'eau peut être ajustée entre 1/99 et 1/1, de préférence entre 1/50 et 1/5.

5. Dispositif de nettoyage selon l'une des revendications précédentes, **caractérisé en ce que** l'eau évacuée par la sortie pour l'eau a une température < 50 °C et/ou est évacuée avec une pression de 0,1 MPa à 0,5 MPa.

6. Dispositif de nettoyage selon l'une des revendications précédentes, **caractérisé en ce que** l'air évacué par la sortie pour l'air a une pression de ≤ 0,5 MPa.

7. Dispositif de nettoyage selon l'une des revendications précédentes, **caractérisé en ce que** les pistolets sous pression sont avec ou sans régulation de pression.

8. Dispositif de nettoyage selon l'une des revendications précédentes, **caractérisé en ce que** chaque pistolet sous pression comprend un bouton électronique qui est positionné sur la tête du pistolet sous pression et que le dispositif est, de préférence, conçu de sorte que le bouton électronique doive être touché une fois pour activer le pistolet sous pression correspondant et une fois de plus pour le désactiver.

9. Dispositif de nettoyage selon l'une des revendications précédentes, **caractérisé en ce que** chaque pistolet sous pression est essentiellement constitué d'aluminium.

10. Dispositif de nettoyage selon l'une des revendications précédentes, **caractérisé en ce que** chaque pistolet sous pression comprend au moins un embout, l'au moins un embout étant, de préférence, constitué d'acier inoxydable et de silicone.

11. Dispositif de nettoyage selon la revendication précédente, **caractérisé en ce que** l'au moins un embout est un embout glissant Luer qui est de préférence conique.

12. Dispositif de nettoyage selon l'une des deux revendications précédentes, **caractérisé en ce que** l'embout est interchangeable et peut, de préférence, être remplacé par un embout avec des applications multiples et/ou avec une fonction de tête de douche intégrée.

13. Dispositif de nettoyage selon l'une des revendications précédentes, **caractérisé en ce qu'**il comprend un écran, de préférence un écran LCD, un écran tactile ou un écran tactile LCD.

14. Dispositif de nettoyage selon l'une des revendications précédentes, **caractérisé en ce qu'**il comprend une pompe péristaltique, de préférence une pompe péristaltique avec un moteur pas à pas.

15. Dispositif de nettoyage selon l'une des revendications précédentes, **caractérisé en ce qu'**il comprend au moins un système de sécurité qui protège contre les défaillances possibles, telles qu'une pénurie d'agent chimique ou d'eau.

16. Utilisation d'un dispositif de nettoyage selon l'une des revendications précédentes dans des processus de préparation de solution, de dosage, de nettoyage et/ou de désinfection dans des établissements de santé, des cliniques, des hôpitaux, des centres de diagnostic et/ou des centres dentaires.
